# FASCICULE DE BREVET EUROPEEN

(11) **EP 0 439 560 B1**
(45) Date de publication et mention de la délivrance du brevet: **18.08.1999**
(21) Numéro de dépôt: 90908544.1
(22) Date de dépôt: 21.05.1990
(51) Int. Cl.: A61L 27/00, A61K 6/00, A61F 2/28, A61C 8/00

(54) **OS ET RACINES DENTAIRES PROTHETIQUES EN NACRE**
KNOCHEN- UND ZAHNWURZELPROTHESEN AUS PERLMUTTER
PROSTHETIC BONES AND DENTAL ROOTS MADE FROM MOTHER OF PEARL

(30) Priorité: 23.05.1989 FR 8907269
(43) Date de publication de la demande: 07.08.1991
(73) Titulaire: CAMPRASSE, Georges, F-77500 Chelles (FR); CAMPRASSE, Serge, F-77500 Chelles (FR)
(72) Inventeur: CAMPRASSE, Georges, F-77500 Chelles (FR); CAMPRASSE, Serge, F-77500 Chelles (FR)
(74) Mandataire: Berger, Helmut
(86) Numéro de dépôt international: FR9000357
(87) Numéro de publication internationale: WO9014111

(56) Documents cités:
- EP-A- 22 724
- EP-A- 230 570
- WO-A-81/02666
- WO-A-87/07826
- DE-A- 2 255 916
- DE-A- 2 421 951
- DE-A- 3 639 030
- DE-B- 1 233 093
- FR-A- 2 361 437
- FR-A- 2 584 290
- US-A- 3 950 850

## Description

La présente invention concerne le remplacement de structures osseuses et radiculo-alvéolaires par des implants, prothèse ou racines dentaires de substitution. Elle a plus particulièrement pour objet un produit de remplacement de ce type qui soit ostéomimétique, ostéoinducteur, histocompatible, qui ne soit pas biodégradable et qui ait une structure telle qu'il ait une élasticité interne comparable à celle de l'os humain ou de la dent naturelle.

On a déjà proposé de nombeux bio-matériaux destinés à remplacer des structures osseuses ou des organes dentaires : mais aucun de ces derniers n'étaient ni ostéomimétique, ni ostéoinducteur, ni bioactif.

Or, on a trouvé et c'est ce qui fait l'objet de l'invention, un produit de remplacement qui remplit les conditions requises à savoir qu'il est histocompatible, ostéomimétique, qu'il est ostéoinducteur, qu'il n'est pas biodégradable et que ses propriétés mécaniques sont constantes et comparables à celles de la dent naturelle et de l'os humain, et qui peut donc convenir parfaitement comme implant ou prothèse osseuse ou racine dentaire de substitution. Selon l'invention le produit de remplacement est réalisé dans la couche nacrée du test de mollusques aquatiques.

Le test de mollusques est composé de deux couches : la couche externe prismatique formée de biocristaux perpendiculaires à la surface qui cristallisent dans un système hexagonal ; la couche interne nacrée, formée de lamelles de biocristaux qui cristallisent selon un système orthorombic. Les 2 couches sont séparées par une membrane de substance organique.

Le procédé selon l'invention utilise la couche interne nacrée du test de mollusques précisément à cause de sa bioarchitecture, de sa composition physicochimique et de son processus de minéralisation.

Le processus physicochimique d'élaboration et de minéralisation de la couche nacrée du test de mollusques, est comparable à celui du tissu osseux et de la racine dentaire naturelle.

C'est la présence dans la substance organique, d'acides aminés tels que : l'acide aspartique, la sérine, l'acide glutamique, la glycine, l'alanine, la leucine, l'arginine, de chondroïtine sulfates, de soufre, de calcium et de magnésium qui confère à la nacre ses propriétés ostéomimétiques et son pouvoir ostéoinducteur. En effet on sait que l'acide aspartique et le soufre jouent un rôle important dans les phénomènes de biominéralisation.

La non biodégradabilité de la nacre provient du diamètre des pores, des surfaces des lamelles de biocristaux qui ont moins de 3 micromètres de diamètre et de profondeur, en forme d'entonnoir et non communiquants. Cette configuration des pores du produit de remplacement lui confère sa microrugosité et sa microporosité indispensable à sa soudure avec le tissu osseux dans lequel il est inséré ; de plus, elle limite la pénétration des néovaisseaux dans le matériau, à la surface. La taille des biocristaux elle-même s'oppose également à la non biodégradabilité du matériau ; en effet l'expérimentation a prouvé que plus un biocristal est gros moins il est biodégradable.

La disposition des lamelles cristallines en mur de briques soudées entre elles par une matrice organique, lui confère des propriétés mécaniques constantes et comparables à celles de la racine dentaire naturelle et de l'os humain, notamment une résistance à la compression de l'ordre de 500 N/mm2, un module d'élasticité en compression de l'ordre de 90 000 N/mm2, une microdureté Vickers HV 0,5 de 190 et une densité de 2,5 proche de celle du tissu osseux et de la dentine naturelle.

De préférence la nacre provient du test de Pinctada, de Tridacne, de la Moule, de la Conque, du Triton, du Buccin, du Nautile.

De préférence, le produit de remplacement se présente sous la forme d'une racine dentaire de substitution, d'une lame, d'une unité radiculo-alvéolaire ou de poudre.

L'invention sera mieux comprise à la lecture de la description de quelques exemples de réalisations.
La figure 1 est une vue de côté d'une racine dentaire de substitution sous forme d'un tenon fileté.
La figure 2 est une vue en perspective d'une unité radiculo-alvéolaire.
La figure 3 est une vue en perspective d'une unité radiculo-alvéolaire en forme de lame.

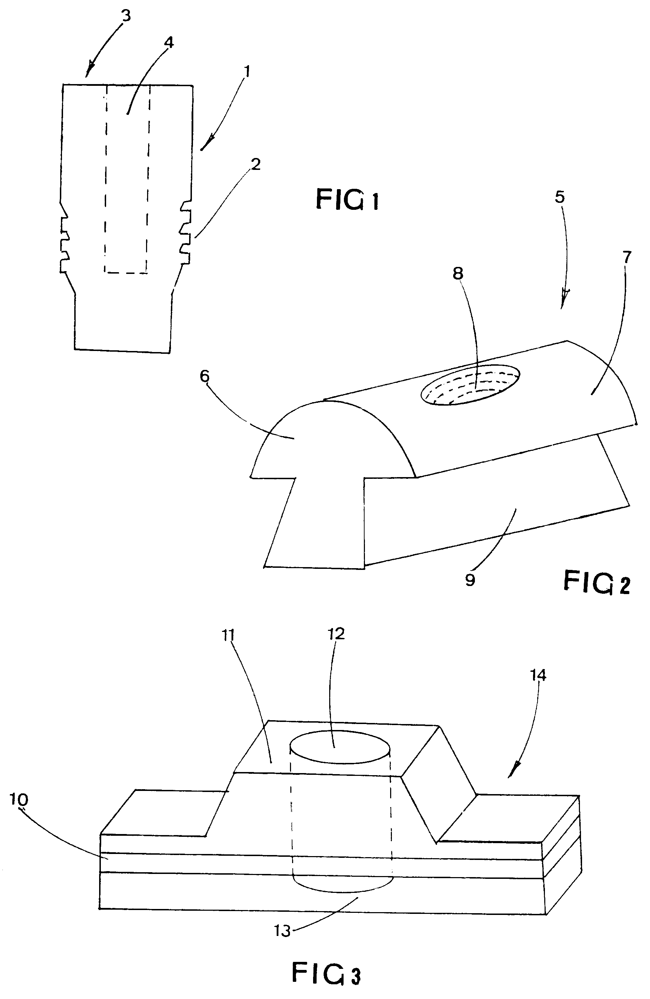

La racine dentaire de substitution montrée à la figure 1 se présente sous la forme d'un tenon cylindroconique 1 présentant un filetage 2 sur une partie de sa hauteur.

La face supérieure 3 du tenon 1 est percée en son centre d'une évidement taraudé 4 dont la profondeur est variable.

Les dimensions sont respectivement de 10 à 14 mm pour la longueur et de 3,45 à 10 mm pour le diamètre.

L'unité radiculo-alvéolaire 5 montrée à la figure 2 comporte une partie semi cylindrique 6 de 13 mm de long, 8 mm de large et 8 mm de haut, dont la face supérieure 7 est convexe et présente en son centre un évidement taraudé 8 sur toute sa hauteur destiné à recevoir le tenon 1 décrit ci-dessus. La partie inférieure 9 a la forme d'une queue d'aronde sur toute la longueur.

L'unité radiculo-alvéolaire 14 montrée à la figure 3 se présente sous la forme d'une lame, c'est à dire d'un élément plat de faible épaisseur. Dans l'exemple illustré, la lame a une épaisseur de 4 mm, une hauteur de 7 mm et comporte sur ses deux faces des sillons parallèles 10. Au centre de la tranche 11 de la lame, constituant la partie supérieure de l'unité 13, on a percé un évidement 12 de 2 mm de diamètre et de 6 mm de profondeur.

Le tenon fileté 1 et les unités radiculo-alvéolaires 5 et 14 décrits ci-dessus ont été réalisés à partir de nacre issue de Pinctada.

Le protocole opératoire relatif à l'insertion de la racine dentaire de remplacement en nacre en forme de tenon est le suivant. Après anesthésie locale ou loco-régionale, incision et décollement de la muqueuse, l'os maxillaire est foré avec des instruments rotatifs calibrés à irrigation interne, refroidis par une solution de chlorure de sodium réfrigérée. L'alvéole artificielle est taraudée avec un instrument calibré, testée avec une jauge. Le tenon 1, stérilisé, maintenu jusqu'alors dans une solution de chlorure de sodium réfrigérée, est vissé et enfoui jusqu'au blocage dans l'alvéole articielle. La muqueuse est suturée.

Le tenon a été inséré selon ce mode opératoire dans le maxillaire humain aussi bien supérieur qu'inférieur. Dans la moitié des cas le tenon 1 a été mis en place sitôt après extraction de la dent, dans l'alvéole déshabitée, dans l'autre moitié chez l'édenté.

L'insertion des unités radiculo-alvéolaires 5 et 14 se fait par enfouissement dans un sillon pratiqué sur la crête maxillaire par la technique de tunnelisation, respectivement de la queue d'aronde 9 et de la lame.

Dans le cas visé ci-dessus, après une période de cicatrisation osseuse variant de 10 à 12 semaines, les produits de remplacement selon l'invention ont reçu une suprastructure supportant une dent artificielle.

L'histobiocompatibilité a été étudiée par prélèvement après 4 mois. On a constaté que le tenon 1 ou l'unité radiculo-alvéolaire 5 n'avait changé ni de forme ni de structure. On a constaté aussi la présence de tissu osseux dans les parties en creux du filetage 2 du tenon 1. De plus l'étude des coupes histologiques a montré une colonisation de surface avec présence d'éléments figurés du sang, essentiellement des hématies, signe d'une micro-circulation a proximité du produit de remplacement en nacre, ainsi que des cellules osseuses jeunes (ostéoblastes) témoignant d'un remaniement osseux, et dans la lumière des pores, des éléments matures (ostéocytes) adhérant fortement par leurs pseudopodes, témoins d'une activité ostéo-génique marquant la véritable ostéo-intégration du produit.

Le matériau sous une autre forme de réalisation a été inséré sous forme plastique à partir d'un mélange sang-poudre dans des cavités kystiques ; dans des foramens réalisés dans la diaphyse fémorale du mouton, dans les cavités sinusiennes. Le matériau sous forme de cylindre a été également inséré sous l'aponévrose et dans le muscle de la région dorsale du mouton.

In vitro des cultures d'ostéoblastes issus d'explants humains ont été mis en présence de fragments de nacre.

D'autre part à partir de nacre on a réalisé des produits de remplacement de pièces osseuses de la région cranio-faciale (os propre du nez, hémi-maxillaire, os de la face, chaîne des osselets de l'oreille interne, des phalanges métatarsiennes et métacarpiennes.

On a aussi réalisé des pièces d'ostéosynthèse en chirurgie traumatologique, du type plaques et vis de fixation.

Bien sûr les formes et dimensions de ces différents produits de remplacement sont fonction de l'application envisagée.

Toutes ces expérimentations ont montré :
a) la parfaite biocompatibilité de la nacre issue du test de mollusques aquatiques bivalves gastéropodes et céphalopodes.
b) la totale pérénnité de structure et de forme du produit de remplacement en nacre, montrant la non biodégradabilité de celle-ci.
c) la parfaite colonisation histochimique de surface du produit de remplacement en nacre, par les cellules osseuses.
d) la parfaite histocompatibilité avec les tissus mous (muqueuse derme, tissu musculaire) avec adhésion histologique en surface des cellules du tissu conjonctif.
e) une excellente adaptation tissulaire et ionique.
f) des propriétés ostéoinductrices aussi bien in vivo qu'in vitro avec accélération de la prolifération des ostéoblastes et une stimulation du processus de minéralisation par maturation des ostéocytes.

L'invention n'est pas limitée aux modes de réalisation qui ont été décrits à titre d'exemples non exhaustifs. Il revient à l'homme du métier de mettre en oeuvre la nacre toutes les fois où il aura besoin d'un produit de remplacement pour une structure osseuse ou une structure radiculo-alvéolaire, dans la forme appropriée à cette utilisation.

## Revendications

1. Produit de remplacement de structure osseuses et radiculo-alvéolaires histo-compatible, ostéomimétique, caractérisé en ce qu'il est réalisé à partir de la couche interne nacrée du test de mollusques aquatiques formées par des lamelles de biocristaux, ce produit étant non biodégradable et ayant des propriétés mécaniques constantes et comparables à celles de l'os humain et de la dent naturelle.

2. Produit selon la revendication 1 caractérisé en ce qu'il est réalisé à partir du test nacré de Pinctada, de Tridacne, d'Huitre, de Moule, de Conque, de Triton, de Buccin, de Nautile.

3. Produit selon l'une des revendications 1 ou 2 caractérisé en ce qu'il est constitué d'une lame (14) découpée en forme de trapèze, dont la tranche supérieure (11) est percée d'un évidement (12) et dont la périphérie comporte des stries longitudinales (10).

4. Produit selon l'une des revendications 1 ou 2, caractérisé en ce qu'il présente la forme d'un tenon cylindroconique (1) présentant un filetage (2) sur une partie de sa hauteur.

5. Produit selon l'une des revendications 1 ou 2, caractérisé en ce qu'il est formé par un corps (5) comprenant une partie supérieure oblongue (6) dont la surface supérieure est convexe et une partie inférieure (9) en forme d'une queue d'aronde s'étendant sur toute la longueur de la partie supérieure.

6. Utilisation de la couche interne nacrée du test nacré de mollusques aquatiques pour la réalisation de produits de remplacement de structures osseuses, histo-compatibles, non biodégradables, ostéomimétiques ayant des propriétés mécaniques constantes, comparables à celles de l'os humain.

7. Utilisation de la couche interne nacrée du test nacré de mollusques aquatiques pour la réalisation de produits de remplacement de structures radiculo-alvéolaires histo-compatibles, ostéomimétiques non biodégradables, ayant des propriétés mécaniques constantes et comparables à celles de la dent naturelle.

## Claims

1. Product as a substitute for osseous and radiculo-alveolar, histologically compatible, osteomimetic structure, characterized in that it is made from the internal nacreous layer of the test of aquatic molluscs formed of lamellae of biocrystals, this product being not biodegradable and having constant mechanical properties comparable with those of the human bone and of the natural tooth.

2. Product according to claim 1, characterized in that it is made from the nacreous test of pinctada, of tridacne, of oyster, of mussel, of conch, of triton, of whelk, of nautilus.

3. Product according to one of claims 1 or 2, characterized in that it consists of a strip (14) cut in the shape of a trapezium of which the upper slice (11) is provided with a bored hollow (12) and of which the periphery comprises longitudinal striae (10).

4. Product according to one of claims 1 or 2, characterized in that it exhibits the shape of a cylindroconical stud (1) formed with a threading (2) over one portion of its height.

5. Product according to one claims 1 or 2, characterized in that it is constituted by a body (5) comprising an upper oblong portion (6) of which the top surface is convex and a lower portion (9) in the shape of a dovetail extending over the whole length of the upper portion.

6. Utilization of the internal nacreous layer of the nacreous test of aquatic molluscs for making products as substitutes of bony, histologically compatible, non biodegradable, osteomimetic structures having constant mechanical properties, comparable with those of the human bone.

7. Utilization of the internal nacreous layer of the nacreous test of aquatic molluscs for making products as substitutes for radiculo-alveolar, histologically compatible, osteomimetic, non biodegradable structures having constant mechanical properties, comparable with those of the natural tooth.

## Patentansprüche

1. Ersatzerzeugnis für wurzelzellige, histologisch kompatibele, knochenmimetische Knochengefüge, dadurch gekennzeichnet, daß es aus der durch Lamellen von Biokristallen gebildeten perlmutterartigen Innenschicht des Testes von Wasserweichtieren hergestellt wird, wobei dieses Erzeugnis nicht biologisch abbaubar ist und mit denjenigen des menschlichen Knochens und des Naturzahnes vergleichbare konstante mechanische Eigenschaften hat.

2. Erzeugnis gemäß Anspruch 1, dadurch gekennzeichnet, daß es aus dem perlmutterartigen Test der Pinctada, der Tridacna, der Auster, der Muschel, der Seemuschel, des Wassermolches, der Buccina, des Nautiluses hergestellt wird.

3. Erzeugnis gemäß einem der Ansprüche 1 oder 2, dadurch gekennzeichnet, daß es aus einem trapezförmig ausgeschnittenen Blatt (14) besteht, dessen obere Abschnitt (11) eine durchgebohrte Aufnahme (12) aufweist und dessen Umfang Längsriefen (10) aufweist.

4. Erzeugnis nach einem der Ansprüche 1 oder 2, dadurch gekennzeichnet, daß es die Gestalt eines zylinder-kegelförmigen, ein Gewinde (2) über einen Teil seiner Höhe aufweisenden Zapfens (1) aufweist.

5. Erzeugnis gemäß einem der Ansprüche 1 oder 2, dadurch gekennzeichnet, daß es durch einen Körper (5) gebildet wird, der einen oberen länglichen Teil (6), dessen obere Fläche konvex ist und einen unteren sich über die ganze Länge des oberen Teiles erstreckenden schwalbenschwanzförmigen Teil (9) umfaßt.

6. Verwendung der perlmutterartigen Innenschicht des perlmutterartigen Testes von Wasserweichtieren für die Herstellung von Ersatzerzeugnissen für histologisch kompatibele, nicht biologisch abbaubare, knochenmimetische Knochengefügen, welche Erzeugnisse mit denjenigen des menschlichen Knochens vergleichbare konstante mechanische Eigenschaften hat.

7. Verwendung der perlmutterartigen Innenschicht des perlmutterartigen Testes von Wasserweichtieren zur Herstellung von Ersatzerzeugnissen für wurzelzellige, biologisch kompatibele, knochenmimetische, nicht biologisch abbaubare Gefügen, welche Erzeugnisse mit denjenigen des Naturzahnes vergleichbare konstante mechanische Eigenschaften haben.
